# EUROPÄISCHE PATENTSCHRIFT

(11) **EP 3 126 034 B1**
(45) Veröffentlichungstag und Bekanntmachung des Hinweises auf die Patenterteilung: **16.10.2019**
(21) Anmeldenummer: 15716451.8
(22) Anmeldetag: 27.03.2015
(51) Int. Cl.: B01D 53/02, B01J 20/12, B01J 20/20, B01J 20/22, B01J 20/28, B01J 20/32

(54) **FILTER UND VERFAHREN ZU DESSEN HERSTELLUNG**
FILTER AND METHOD FOR PRODUCING SAME
FILTRE ET PROCÉDÉ DE PRODUCTION ASSOCIÉ

(30) Priorität: 31.03.2014 DE 102014206081
(43) Veröffentlichungstag der Anmeldung: 08.02.2017
(73) Patentinhaber: Lufthansa Technik AG, 22335 Hamburg (DE)
(72) Erfinder: PAUL, Manfred, 55299 Nackenheim (DE); LUKA, Patrick, 64546 Mörfelden-Waldorf (DE); SWEREDJUK,Robert, 87463 Dietmannsried-Reicholzried (DE); POMMERSHEIM,Rainer, 55116 Mainz (DE)
(74) Vertreter: Glawe, Delfs, Moll
(86) Internationale Anmeldenummer: PCT/EP2015/056799
(87) Internationale Veröffentlichungsnummer: WO 2015/150288

(56) Entgegenhaltungen:
- DE-A1- 4 200 995
- DE-A1-102012 202 563
- DE-U1-202010 009 413

## Beschreibung

Die Erfindung betrifft einen Filter zum Binden von Bestandteilen eines Gasstroms, mit einem Trägerkörper und einer auf die Oberflächen des Trägerkörpers aufgebrachten Filterschicht. Gegenstand der Erfindung ist ferner ein Verfahren zur Herstellung eines solchen Filters, die Verwendung eines solchen Filters zur Filterung der Atemluft in Verkehrsmitteln, insbesondere Flugzeugen, sowie ein mit einem erfindungsgemäßen Filter ausgerüstetes Flugzeug.

Viele Verkehrsmittel, insbesondere Flugzeuge und beispielsweise Hochgeschwindigkeitszüge, werden in der Regel druckdicht gebaut und künstlich belüftet. Flugzeuge weisen eine Druckkabine auf, in der in Reiseflughöhe ein über dem Außenluftdruck liegender Kabinendruck eingestellt wird.

Die Luftversorgung einer Druckkabine erfolgt in der Regel sowohl durch Umwälzen und Reinigen von Kabinenluft als auch durch Zumischen von außen zugeführter Frischluft. Da der Außendruck in Reiseflughöhe unter dem Kabinendruck liegt, muss die zuzuführende Frischluft verdichtet werden. In der Regel wird für die Frischluftzufuhr ein Teil des Luftstroms aus dem Verdichter eines oder mehrerer Flugzeugtriebwerke (sogenannte Zapfluft) abgezweigt, auf ein gewünschtes Temperaturniveau gekühlt und der Kabinenluft beigemischt.

Die Zapfluft von Strahltriebwerken kann mit Ölrückständen oder Ölnebeln verunreinigt sein, insbesondere dann, wenn beispielsweise Schmieröl des Triebwerks im Bereich der Welle oder dergleichen austritt und von dem Verdichterluftstrom mitgerissen wird. Die Öle von Strahltriebwerken können für den Menschen schädliche Bestandteile oder Additive wie beispielsweise Trikresylphosphat (TCP) enthalten. Mitgerissene Ölrückstände können ferner zu einem unangenehmen Ölgeruch in der Flugzeugkabine führen. Umgewälzte Kabinenluft kann ebenfalls Geruchs- oder Schadstoffe enthalten.

Im Stand der Technik ist es daher bereits bekannt, Filtersysteme für die Zapfluft von Triebwerken und/oder umgewälzte Kabinenluft vorzusehen. Zum einen sind aus offenkundiger Vorbenutzung beispielsweise Aktivkohlefilter bekannt. Diese besitzen nur ein relativ geringes Aufnahmevermögen und binden Schadstoffe durch Physisorption reversibel, sodass diese bei höherer Belastung eines Aktivkohlefilters auch wieder freigesetzt werden können.

Bekannt ist ferner, unerwünschte Bestandteile aus der Kabinenluft durch katalytische Oxidation zu entfernen (beispielsweise US 2003/0188850 A1, US 2009/0227195 A1, US 2010/0158775 A1 und US 2005/0053515 A1). Solche katalytischen Systeme sind sehr aufwändig in der Installation und im Betrieb, da ständig Betriebsbedingungen gewahrt sein müssen, die eine katalytische Oxidation der Schadstoffe ermöglichen, beispielsweise Temperaturen von über 200° C für eine thermische Katalyse oder Zufuhr von UV-Strahlung für eine Photokatalyse. WO 2013/124168 A1 offenbart ein Filtergranulat zum Filtern der Atemluft in Verkehrsflugzeugen.

Der Erfindung liegt die Aufgabe zugrunde, eine einfach zu handhabende und wirksame Möglichkeit zur Entfernung von Schadstoffen wie beispielsweise Ölrückständen aus einem Gasstrom, insbesondere der Zapfluft oder Kabinenluft von Flugzeugen, zur Verfügung zu stellen, die sich einfach und ohne größere Probleme in bestehende Systeme integrieren lässt.

Gelöst wird diese Aufgabe durch einen Filter zum Binden von Bestandteilen eines Gasstroms, mit einem Trägerkörper, der eine Zelldichte von 50 bis 1.600 cpsi aufweist, und einer auf die Oberflächen des Trägerkörpers aufgebrachten Filterschicht, wobei die Filterschicht folgende Komponenten aufweist:
a) eine Komponente zur Physisorption von Bestandteilen,
b)eine Komponente zur Chemisorption von Bestandteilen,
c)eine Komponente zur Lösung von Ölbestandteilen, die ionische Flüssigkeiten enthält.

Gegenstand der Erfindung sind ferner ein Verfahren zur Herstellung eines erfindungsgemäßen Filters, die Verwendung eines solchen Filters zur Filterung der Atemluft in Verkehrsmitteln, insbesondere Flugzeugen; sowie Flugzeuge, die mit erfindungsgemäßen Filtern ausgerüstet sind.

Zunächst seien einige im Rahmen der Erfindung verwendete Begriffe erläutert.

Der Begriff Trägerkörper bezeichnet ein von einem Gasstrom überströmbares bzw. durchströmbares Substrat, dessen Oberflächen wenigstens teilweise die unten näher beschriebene Filterschicht tragen können. Geeignete Trägerkörper sind beispielsweise aus der Kraftfahrzeugtechnik bekannte Trägerkörper für Abgaskatalysatoren. Solche Trägerkörper weisen eine bezogen auf ihr Volumen hohe Oberfläche kombiniert mit einem geringen Durchflusswiderstand auf, der eine Durchströmung mit großen Gasmengen möglich macht.

Auf Oberflächen des Trägerkörpers ist eine Filterschicht aufgebracht. Diese tritt in Wechselwirkung mit vorbeiströmenden Gas und übt die gewünschte Filterwirkung aus. Art und Dicke der Filterschicht sind so bemessen, dass die erforderliche Durchströmbarkeit des Filters gewährleistet bleibt.

Erfindungsgemäß sind in der Filterschicht drei Komponenten enthalten. Der Begriff Komponente ist in diesem Kontext funktional zu verstehen und bedeutet Teile oder Bestandteile der Schicht, die die beschriebene Funktion ausüben können.

Eine erste Komponente dient zur Physisorption von Bestandteilen des Gasstroms. Bei der Physisorption wird ein absorbiertes Molekül durch in der Regel vergleichsweise schwache physikalische Kräfte (Bindungsenergien häufig im Bereich 4-40 kJ/mol) an der Oberfläche der Komponente (des physisorbierenden Substrats) gebunden. Die Physisorption führt in der Regel zu keiner chemischen Veränderung der absorbierten Stoffe und ist üblicherweise reversibel.

Eine weitere Komponente dient zur Chemisorption von Bestandteilen des Gasstroms. Bei der Chemisorption wird das Absorbat (der gebundene Bestandteil des Gasstroms) und/oder das Absorbens (die Komponente, die das Substrat für die Chemisorption bildet) chemisch verändert. Bindungsenergien können beispielsweise im Bereich um 800 kJ/mol liegen. Die Chemisorption ist in der Regel irreversibel, d.h. die chemisorbierten Bestandteile sind dauerhaft gebunden.

Erfindungsgemäß dient eine dritte Komponente zur Lösung von Ölbestandteilen. Der Begriff Ölbestandteile bezeichnet im Gasstrom mitgeführte Bestandteile beispielsweise vom Schmieröl eines Strahltriebwerks, in der Regel liegen solche Ölbestandteile als Aerosol fein verteilt in dem Gasstrom vor. Die genannte Komponente enthält ein Lösemittel, das Ölbestandteile in Lösung überführen und damit aus dem Gasstrom entfernen kann. Der Dampfdruck des Lösemittels und der daraus hervor gehenden Lösung der Ölbestandteile ist erfindungsgemäß so gering, dass unter den Betriebsbedingungen des entsprechenden Filters Lösemittel und/oder Lösung nicht in nennenswertem Maße in den Gasstrom eingetragen werden. Als Lösemittel werden die unten näher beschriebenen ionischen Flüssigkeiten verwendet.

Der erfindungsgemäße Filter vereint somit drei Komponenten und damit auch drei Funktionen. Die Komponente zur Physisorption kann erfindungsgemäß beispielsweise ausgewählt sein aus der Gruppe bestehend aus Aktivkohle, Kieselerden, Zeolithen und Bentonit und bewirkt eine schnelle und wirksame Abreicherung von der Physisorption zugänglichen Bestandteilen des Gasstroms wie beispielsweise Luftschadstoffen, Gerüchen oder dergleichen.

Die chemisorbierende Komponente erlaubt eine dauerhafte und damit irreversible Bindung entsprechender unerwünschter Bestandteile des Gasstroms. Es ist im Rahmen der Erfindung auch möglich, dass Bestandteile des Gasstroms zunächst physisorbiert und damit im Bereich des Filters lokalisiert werden und anschließend eine Chemisorption und damit dauerhafte Bindung erfolgt.

Die Komponente zur Lösung von Ölbestandteilen erlaubt eine dauerhafte und sichere Entfernung entsprechender Ölaerosole aus einem Gasstrom, damit wird wirkungsvoll dem Eintrag unerwünschter Ölgerüche sowie Schadstoffe aus Schmieröl in die Kabinenluft von den Flugzeugen vorgebeugt. Die Entfernung dieser Ölbestandteile durch Lösen in einem Lösungsmittel bindet die Ölbestandteile dauerhaft und besitzt zudem eine hohe Aufnahmekapazität.

Der erfindungsgemäße Filter erlaubt somit eine sichere und schnelle, aber auch dauerhafte und irreversible Entfernung typischer Geruchs- und Schadstoffbestandteile aus einem Gasstrom, insbesondere der Atemluft einer Flugzeugkabine.

Ein wesentliches Merkmal der Erfindung ist es, dass die genannten Komponenten Bestandteil einer Filterschicht auf einem Trägerkörper sind. Die Erfindung hat erkannt, dass die Ausgestaltung des Filters als Trägerkörper mit darauf aufgebrachter Filterschicht die Schaffung eines Filters ermöglicht, der einerseits einem hohen Gasdurchsatz mit guter Filterwirkung erlaubt und andererseits einen geringen Strömungswiderstand aufweist, so dass er ohne weiteres und insbesondere ohne zusätzliche Maßnahmen in bestehende Systeme, beispielsweise Systeme zur Versorgung einer Flugzeugkabine mit Atemluft, integrierbar ist. Insbesondere ist es erfindungsgemäß in der Regel nicht erforderlich, Maßnahmen wie beispielsweise Druckerhöhung oder erhöhte Pumpleistung bei der Atemluftversorgung vorzusehen, um einen großen Strömungswiderstand eines Filters auszugleichen.

Der Trägerkörper ist erfindungsgemäß bevorzugt ausgewählt aus der Gruppe bestehend aus keramischen Trägerkörpern und Metallträgerkörpern. Trägerkörper aus Kunststoff können ebenfalls Verwendung finden. Es können insbesondere Trägerkörper verwendet werden, wie sie als Katalysatorträger in der Abgastechnik von Kraftfahrzeugen Verwendung finden. Trägerkörper aus Metall können beispielsweise Hohlräume bzw. Zellen in der Form von Honigwaben aufweisen, geeignete Trägerkörper sind beispielsweise in WO 2010/108755 A1 beschrieben. Kommerziell erhältlich sind geeignete Trägerkörper beispielsweise unter der Bezeichnung METALIT® von der Emitec Gesellschaft für Emissionstechnologie GmbH.

Erfindungsgemäß sind die Zelldichten der verwendeten Trägerkörper im Bereich 50 bis 1.600 cpsi (cells per square inch), bevorzugt sind Bereiche von 100 bis 1.000 cpsi und 150 bis 500 cpsi. Die Stärke der Zellwände liegt bevorzugt im Bereich 10-100 µm, weiter vorzugsweise 10-50 µm. Die Form der Zellen im Querschnitt (Schnittebene senkrecht zur Strömungsrichtung) kann beispielsweise sinusförmig, quadratisch, dreieckig oder hexagonal sein.

Der erfindungsgemäße Trägerkörper kann einen Mantel aufweisen, der beispielsweise eine Wandstärke von 0,5-5 mm, weiter vorzugsweise 1-2 mm aufweisen kann. Der Mantel dient der Stabilisierung des Trägerkörpers. Alternativ ist es möglich, auf einen Mantel zu verzichten und den Trägerkörper im Wesentlichen formschlüssig in einen Strömungskanal einzuführen, dessen Wände den Trägerkörper stabilisieren. Die Länge eines Trägerkörpers in Strömungsrichtung kann erfindungsgemäß beispielsweise 10-1000 mm, vorzugsweise 10-100 mm betragen. Der durch den erfindungsgemäßen Filter bewirkte Druckverlust beträgt bevorzugt 10 mbar oder weniger, weiter vorzugsweise 5 mbar oder weniger.

Die Komponente zur Physisorption kann ausgewählt sein aus der Gruppe bestehend aus Aktivkohle, Bentonit, Kieselerden und Zeolithen.

Die Komponente zur Chemisorption kann erfindungsgemäß geeignete Aminosäuren oder Aminosäuresequenzen, insbesondere Peptide oder Proteine enthalten. Peptide sind in der Regel kurzkettiger als Proteine, zwischen den beiden Begriffskategorien gibt es einen fließenden Übergang. Solche Peptide oder Proteine können durch Chemisorption Schadstoffe binden, die sogenannte proteinreaktive Substanzen sind, also Verbindungen, die mit Proteinen oder Proteinderivaten der Komponente zur Chemisorption reagieren und eine chemische Bindung eingehen. Darunter fallen typische Luftschadstoffe wie Aldehyde (insbesondere Formaldehyd) und viele flüchtige organische Verbindungen (volatile organic components VOC). Geeignete Aminosäurensequenzen lassen sich beispielsweise durch Hydrolyse von Eiweißen wie beispielsweise Skleroproteinen herstellen, ferner kann man beispielsweise keratinhaltige Fasern wie beispielsweise Schafwollfasern verwenden.

Erfindungsgemäß enthält die Komponente zur Lösung von Ölbestandteilen ionische Flüssigkeiten. Ionische Flüssigkeiten sind Salze, die bei den Betriebstemperaturen des Filters in der Regel flüssig sind. Typischerweise sind ionische Flüssigkeiten bei Temperaturen unter 100° C flüssig, bevorzugt auch bei Raumtemperatur. Ionische Flüssigkeiten besitzen einen sehr niedrigen, kaum messbaren Dampfdruck und verfügen über gute Lösungseigenschaften für Ölbestandteile.

Bevorzugt sind in erfindungsgemäßen ionischen Flüssigkeiten die Kationen ausgewählt aus der Gruppe bestehend aus ggf. alkylierten Imidazolium-, Pyridinium-, Pyrrolidinium-, Guanidinium-, Uronium-, Thiouronium-, Piperidinium-, Morpholinium-, Ammonium- und Phosphoniumionen und die Anionen ausgewählt aus der Gruppe bestehend aus Tetrafluoroboraten, Trifluoracetaten, Triflaten, Hexafluorophosphaten, Phosphinaten, Tosylaten, Imiden, Amiden, Sulfaten und Halogeniden.

Bei einer vorteilhaften Ausführungsform der Erfindung weist die Filterschicht eine Matrix auf. Der Begriff Matrix bezeichnet eine Substanz, die als Strukturbildner dient und die beschriebenen Komponenten trägt. Bestandteile der Matrix können Komponenten zur Physisorption und/oder Chemisorption umfassen. Die Matrix kann ferner Bindemittel enthalten, beispielsweise Bindemittel ausgewählt aus der Gruppe bestehend aus Mannuron-, Guluron-, Alginat- und Pektinsalzen. Diese Bindemittel können beispielsweise als Alkalisalze zunächst in wässriger Lösung zur Verfügung gestellt und mit weiteren Komponenten vermischt werden. Durch Austausch der Alkalionen gegen beispielsweise Erdalkalionen (insbesonder Ca²⁺-Ionen) kann man aus den löslichen Salzen unlösliche Salze machen, die damit ausfallen oder gelieren und so entsprechende Filterschichten lokalisieren bzw. fixieren. Erfindungsgemäß ist es ebenfalls möglich, die Gelierung durch geeignte organische Kationen zu bewirken, beispielsweise oligomere oder polymere Kationen wie PEI (Polyethylenimnine), PDMDAAC (Poly (dimethyldiallylammoniumchlorid) oder PLL (Poly-L-Lysin). Überraschenderweise hat die Erfindung erkannt, dass auch Kationen der verwendeten ionischen Flüssigkeit zur Gelierung verwendet werden bzw. dazu beitragen können.

Eine solche Matrix mit den genannten Bindemitteln kann auch ionische Flüssigkeiten einschließen bzw. einkapseln. Die Verkapselung von Ölen in einer Matrix aus einem Alkalimetallalginat ist bspw. beschrieben in US 4,389,419. Diese Schrift wird durch Bezugnahme darauf zum Gegenstand der vorliegenden Offenbarung gemacht.

Gegenstand der Erfindung ist ferner ein Verfahren zur Herstellung eines erfindungsgemäßen Filters, gekennzeichnet durch folgende Schritte:
a) Bereitstellen eines Trägerkörpers,
b) Beschichten der Oberflächen des Trägerkörpers mit der Filterschicht.

Bevorzugt werden die Oberflächen des Trägerkörpers mit einer Lösung von Bestandteilen der Filterschicht beschichtet. Dies kann weiter bevorzugt durch Eintauchen des Trägerkörpers in eine solche Lösung oder Besprühen mit der Lösung erfolgen. Nachdem Beschichten kann bevorzugt eine Fixierung erfolgen. Dies geschieht bevorzugt durch Verfestigen eines Bindemittels der Filterschicht. Das Verfestigen kann durch Einwirkung von Temperatur oder bevorzugt durch chemische Reaktionen erfolgen. Insbesondere kann die Reaktion ausgelöst werden durch Aufbringen einer weiteren Komponente. Sind die Bindemittel beispielsweise ausgewählt aus der Gruppe bestehend aus Mannuron-, Guluron-, Alginat- und Pektinsalzen, können sie beispielsweise als Alkalisalze zunächst in wässriger Lösung zur Verfügung gestellt und mit weiteren Komponenten vermischt werden. Diese wässrige Lösung wird auf die Oberflächen des Trägerkörpers aufgebracht, beispielsweise durch Eintauchen. Durch Austausch der Alkalionen gegen beispielsweise Erdalkalionen kann man aus den löslichen Salzen unlösliche Salze machen, die damit ausfallen oder gelieren und so entsprechende Filterschichten lokalisieren bzw. fixieren. Dies kann erfolgen durch Eintauchen in oder Aufsprühen von einer Lösung von Erdalkaliionen.

Ein weiterer Gegenstand der Erfindung ist die Verwendung eines erfindungsgemäßen Filters zur Filterung der Atemluft in Verkehrsmitteln, insbesondere Flugzeugen. Erfindungsgemäß kann die Filterung von Zapfluft erfolgen, bevor diese erstmalig dem Klimatisierungssystem der Kabine zugeleitet wird, um Ölrückstände oder andere aus dem Verdichtertrakt des Triebwerks stammende Schadstoffe zu entfernen. Alternativ oder zusätzlich kann umgewälzte Kabinenluft gefiltert werden, um auch sonstige Luftschadstoffe, Gerüche oder dergleichen zu entfernen.

Gegenstand der Erfindung ist ferner ein Flugzeug, das wenigstens einen erfindungsgemäßen Filter enthält. Die Filter können zur Reinigung von Zapfluft oder umgewälzter Kabinenluft verwendet werden und an einem oder mehreren der folgenden Einbauorte angeordnet sein:
- zwischen Zapfluftventil und Kühleinheit,
- zwischen Kühl- und Mischeinheit,
- zwischen Mischeinheit und Austritt der Versorgungsrohre in Kabine und/oder Cockpit.

Die verwendeten Begriffe werden nachfolgend im Zusammenhang mit dem Ausführungsbeispiel erläutert. Bevorzugt ist die Anordnung innerhalb der Druckkabine vor dem Austritt der Versorgungsrohre in Kabine und/oder Cockpit.

Ausführungsbeispiele der Erfindung werden nachfolgend beschrieben. In den Figuren zeigen:
- Fig. 1:: Schematisch einen erfindungsgemäßen Filter;
- Fig. 2:: Schematisch das Klimasystem eines Verkehrsflugzeugs.

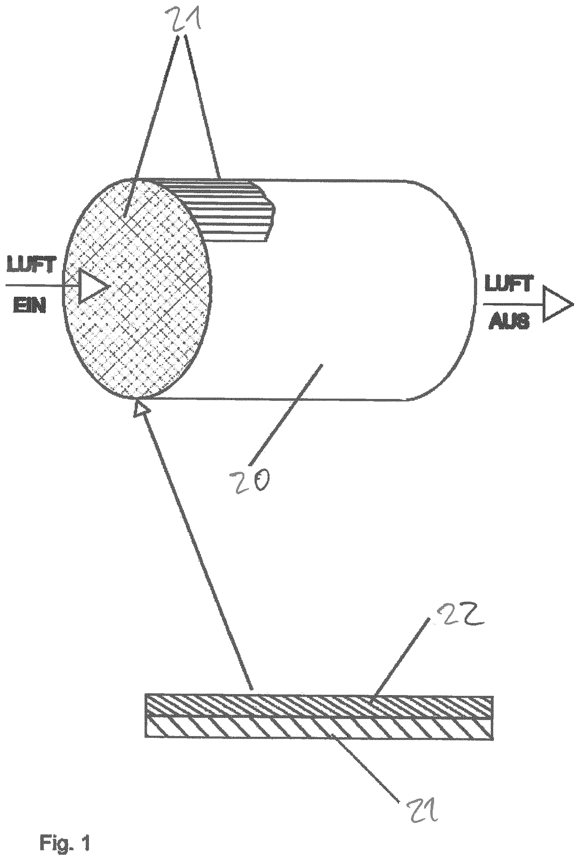

Figur 1 zeigt schematisch einen erfindungsgemäßen Filter 20. In seinem Inneren befindet sich ein Trägerkörper 21 mit einer Struktur in der Art von Honigwaben. Wie im unteren Detailausschnitt der Figur 1 erkennbar, ist auf der Oberfläche des Trägerkörpers 21 eine Filterschicht 22 aufgetragen. In diesem Ausführungsbeispiel weist der Filter eine Zelldichte von 200 cpsi auf. Die Zellwände bestehen aus einer 40 µm starken Metalllegierungsfolie aus dem Material DIN 1.4767 (mit Yttrium und Hafnium legierter aluminiumhaltiger ferritischer Chromstahl). Der Mantel des Filters weist eine Stärke von 1,5 mm auf, das Material des Mantels ist eine Metalllegierung DIN 1.4509 (nichtrostender ferritischer Chromstahl). Die Länge des Mantels beträgt 84,5 mm, der Mantelüberstand zur Filtermatrix an jedem Längsende 5 mm.

Die Herstellung eines erfindungsgemäßen Filters wird nachfolgend beschrieben.

### Beispiel 1

In 900 ml Wasser werden folgende Bestandteile gelöst bzw. dispergiert:
- 80 - 100, bevorzugt 90 g Bentonit
- 30 - 60, bevorzugt 45 g Aktivkohle
- 20 - 50, bevorzugt 35 g Ethyl-Methylimidazolium-Ethylsulfat
- 2 - 8, bevorzugt 5 g N-(L-α-Aspartyl)-L-phenylalanin-methylester
- 4 - 8, bevorzugt 6 g Natriumalginat.

Die so hergestellte Lösung bzw. Dispersion wird auf den Trägerkörper 21 aufgebracht, bevorzugt wird der Trägerkörper in diese Lösungen eingetaucht. Der so vorbehandelte Trägerkörper wird anschließend in eine 2%ige wässrige Calciumchloridlösung eingetaucht. Durch den Zutritt von Calcium (oder anderen geeigneten mehrwertigen Metallkationen) entstehen unlösliche Alginatsalze, die die Filterschicht zum Gelieren und zum Erstarren bringen. Anschließend wird im Luftstrom getrocknet. Im nächsten Schritt wird auf die fixierte Filterschicht zusätzlich ionische Flüssigkeit aufgetragen. Zu diesem Zweck wird der Trägerkörper in 1-Ethyl-2-Methylimidazolium-Ethylsulfat eingetaucht oder diese ionische Flüssigkeit wird aufgesprüht und im Luftstrom getrocknet.

### Beispiel 2

Wie in Beispiel 1 wird die dort beschriebene Lösung bzw. Mischung hergestellt und auf die Oberflächen des Trägerkörpers aufgebracht. Die Fixierung dieser Filterschicht erfolgt jedoch im zweiten Schritt unmittelbar durch Eintauchen des Trägerkörpers in 1-Ethyl-2-Methylimidazolium-Ethylsulfat und anschließende Trocknung im Luftstrom. Bei dieser Variante der Erfindung wird also in einem einzigen Schritt die Fixierung bzw. Gelierung der Filterschicht durchgeführt und die ionische Flüssigkeit aufgetragen.

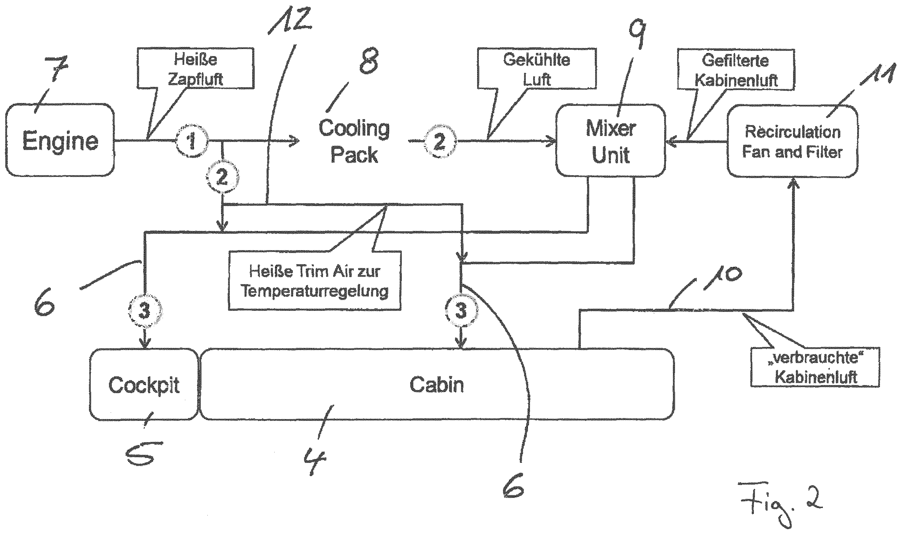

Figur 2 zeigt schematisch das Klimasystem eines Verkehrsflugzeugs. Eine Kabine 4 und ein Cockpit 5 eines Flugzeugs werden von Versorgungsrohren 6 mit Luft gespeist. Diese Luft ist ein Gemisch aus temperierter Frischluft und gefilterter, umgewälzter Kabinenluft.

Die Frischluft wird durch Abzweigen so genannter Zapfluft aus dem Verdichter eines oder mehrerer Triebwerke 7 bereitgestellt. Der Hauptteil der aufgrund der Verdichtung erwärmten Zapfluft (Temperatur typischerweise 215-260 °C) wird einer Kühleinheit 8 zugeführt und auf die gewünschte Temperatur gekühlt. Der gekühlte Luftstrom wird einer Mischeinheit 9 zugeführt.

Verbrauchte Kabinenluft wird mittels einer Leitung 10 aus der Kabine abgesaugt und in einer Vorrichtung 11 zum Umwälzen und Filtern der Kabinenluft gereinigt. Diese Vorrichtung 11 arbeitet nach dem Stand der Technik. In der Mischeinheit 9 wird gereinigte, umgewälzte Kabinenluft und gekühlte Frischluft in einem vorgegebenen Verhältnis gemischt und über die Versorgungsrohre 6 dem Cockpit und der Kabine zugeführt. Zur Regelung der Temperatur in der Kabine kann über die Leitung 12 ein Teil der heißen, noch nicht gekühlten Zapfluft abgezweigt und mit dem von der Mischeinheit 9 zu Cockpit 5 und Kabine 4 geführten Luftstrom vermischt werden.

Die erfindungsgemäßen Filter können in einem solchen Klimasystem an verschiedenen Stellen verbaut werden, die in der Figur mit den Bezugsziffern 1, 2 und 3 gekennzeichnet sind.

Bei dem Einbauort 1 wird die noch heiße Zapfluft unmittelbar nach ihrer Entnahme aus dem Triebwerk 7 gefiltert. Dieser Einbauort hat den Vorteil, dass etwaige in der Zapfluft vorhandene Ölrückstände unmittelbar nach dem Austritt aus dem Triebwerk gefiltert werden und nicht in das Klimasystem gelangen können. Nachteilig an diesem Standort sind insbesondere die erschwerten Betriebsbedingungen durch hohe Temperatur, hohe Strömungsgeschwindigkeit und hohen Druck, die den effizienten Betrieb eines erfindungsgemäßen Filters erschweren.

Bei dem möglichen Einbauort 2 wird einerseits die bereits gekühlte Zapfluft und andererseits der für die Zwecke der Temperaturregelung abgezweigte heiße Teil der Zapfluft mittels jeweils wenigstens eines erfindungsgemäßen Filters gereinigt. Problematisch ist hier insbesondere die Tatsache, dass zwei Luftströme mit insbesondere sehr unterschiedlichen Temperaturen gefiltert werden müssen.

Bevorzugt ist es, erfindungsgemäße Filter am Einbauort 3 vorzusehen und die bereits temperierte Luft unmittelbar vor ihrer Zufuhr zu Cockpit 5 bzw. Kabine 4 zu filtern.

Dieser Einbauort 3 hat verschiedene Vorteile. Die Filter werden an diesem Einbauort von verhältnismäßig kühler Luft mit wenig variierender Temperatur durchströmt. Die Temperatur des Luftstroms am Einbauort 3 liegt in jedem Fall unter 100 °C und erlaubt daher eine verhältnismäßig einfache weil nicht notwendigerweise temperaturfeste Konstruktion des Filters. Der Querschnitt der Versorgungsrohre 6 ist in der Regel groß (typische Durchmesser beispielsweise 150-164 mm), dies erlaubt einen entsprechend großen und damit wirkungsvollen Querschnitt des Filters, der zudem von wenig komprimierter Luft mit geringer Strömungsgeschwindigkeit durchströmt wird. Der Einbauort 3 ist zudem für Wartungszwecke gut zugänglich, da er sich innerhalb der Druckkabine befindet, beispielsweise ist ein Zugang über entsprechende Öffnungen im vorderen Frachtraum eines Flugzeugs möglich. Abhängig vom Flugzeugtyp kann die Zahl der erforderlichen Einbauorte 3 variieren. Bei einem kommerziellen Verkehrsflugzeug der A320-Familie werden beispielsweise fünf Filter benötigt, für eine Boeing 737 drei Filter.

## Patentansprüche

1. Filter (20) zum Binden von Bestandteilen eines Gasstroms, mit einem Trägerkörper (21), der eine Zelldichte von 50 bis 1.600 cpsi aufweist, und einer auf die Oberflächen des Trägerkörpers (21) aufgebrachten Filterschicht (22), **dadurch gekennzeichnet, dass** die Filterschicht (22) folgende Komponenten aufweist:
a) eine Komponente zur Physisorption von Bestandteilen,
b) eine Komponente zur Chemisorption von Bestandteilen,
c) eine Komponente zur Lösung von Ölbestandteilen, die ionische Flüssigkeiten enthält.

2. Filter (20) nach Anspruch 1, **dadurch gekennzeichnet, dass** der Trägerkörper (21) ausgewählt ist aus der Gruppe bestehend aus keramischen Trägerkörpern und Metallträgerkörpern.

3. Filter (20) nach Anspruch 1 oder 2, **dadurch gekennzeichnet, dass** der Trägerkörper (21) eine Zelldichte von 100 bis 1.000 cpsi, vorzugsweise 150 bis 500 cpsi aufweist.

4. Filter (20) nach einem der Ansprüche 1 bis 3, **dadurch gekennzeichnet, dass** er unter Anwendungsbedingungen einen Druckabfall von 10 mbar oder weniger, vorzugsweise 5 mbar oder weniger aufweist.

5. Filter (20) nach einem der Ansprüche 1 bis 4, **dadurch gekennzeichnet, dass** die Komponente zur Physisorption ausgewählt ist aus der Gruppe bestehend aus Aktivkohle, Bentonit, Kieselerden und Zeolithen; und/oder dass die Komponente zur Chemisorption Peptide, Proteine oder keratinhaltige Fasern enthält.

6. Filter (20) nach einem der Ansprüche 1 bis 5, **dadurch gekennzeichnet, dass** die ionischen Flüssigkeiten Kationen ausgewählt aus der Gruppe bestehend aus ggf. alkylierten Imidazolium-, Pyridinium-, Pyrrolidinium-, Guanidinium-, Uronium-, Thiouronium-, Piperidinium-, Morpholinium-, Ammonium- und Phosphoniumionen und Anionen ausgewählt aus der Gruppe bestehend aus Tetrafluoroboraten, Trifluoracetaten, Triflaten, Hexafluorophosphaten, Phosphinaten, Tosylaten, Imiden, Amiden, Sulfaten und Halogeniden enthalten.

7. Filter (20) nach einem der Ansprüche 1 bis 6, **dadurch gekennzeichnet, dass** die Filterschicht (22) eine Matrix aufweist, wobei Bestandteile der Matrix vorzugsweise Komponenten zur Physisorption und/oder Komponenten zur Chemisorption umfassen; wobei die Matrix vorzugsweise Bindemittel enthält; wobei die Bindemittel vorzugsweise ausgewählt sind aus der Gruppe bestehend aus Mannuron-, Guluron-, Alginat- und Pektinsalzen.

8. Filter (20) nach Anspruch 7, **dadurch gekennzeichnet, dass** die Matrix ionische Flüssigkeiten einschließt.

9. Verfahren zur Herstellung eines Filters (20) nach einem der Ansprüche 1 bis 8, **gekennzeichnet durch** folgende Schritte:
c) Bereitstellen eines Trägerkörpers (21),
d) Beschichten der Oberflächen des Trägerkörpers (21) mit der Filterschicht (22).

10. Verfahren nach Anspruch 9, **dadurch gekennzeichnet, dass** die Oberflächen des Trägerkörpers (21) mit einer Lösung von Bestandteilen der Filterschicht (22) beschichtet werden; wobei vorzugsweise nach dem Beschichten mit einer Lösung von Bestandteilen der Filterschicht (22) eine Fixierung erfolgt; wobei vorzugsweise das Fixieren durch Verfestigen eines Bindemittels der Filterschicht (22) erfolgt; wobei vorzugsweise das Verfestigen des Bindemittels durch eine Reaktion mittels Aufbringen einer weiteren Komponente erfolgt.

11. Verwendung eines Filters (20) nach einem der Ansprüche 1 bis 8 zur Filterung der Atemluft in Verkehrsmitteln, insbesondere Flugzeugen.

12. Verwendung eines Filters (20) nach einem der Ansprüche 1 bis 8 zur Filterung der Zapfluft aus dem Verdichter eines Flugzeugtriebwerks.

13. Flugzeug, **dadurch gekennzeichnet, dass** es wenigstens einen Filter (20) nach einem der Ansprüche 1 bis 8 zur Filterung der Atemluft aufweist.

14. Flugzeug nach Anspruch 13, **dadurch gekennzeichnet, dass** wenigstens ein Filter (20) an einem oder mehreren der folgenden Einbauorte (1, 2, 3) angeordnet ist:
- zwischen Zapfluftventil und Kühleinheit (8),
- zwischen Kühl- und Mischeinheit (9),
- zwischen Mischeinheit (9) und Austritt der Versorgungsrohre (6) in Kabine (4) und/oder Cockpit (5).

15. Flugzeug nach Anspruch 14, **dadurch gekennzeichnet, dass** wenigstens ein Filter (20) innerhalb der Druckkabine vor dem Austritt der Versorgungsrohre (6) in Kabine (4) und/oder Cockpit (5) angeordnet ist.

## Claims

1. Filter (20) for binding constituents of a gas stream, having a supporting member (21) which has a cell density of from 50 to 1600 cpsi and a filter layer (22) applied to the surfaces of the supporting member (21), **characterized in that** the filter layer (22) has the following components:
a) a component for the physisorption of constituents,
b) a component for the chemisorption of constituents,
c) a component for dissolving oil constituents which comprises ionic liquids.

2. Filter (20) according to Claim 1, **characterized in that** the supporting member (21) is selected from the group consisting of ceramic supporting members and metal supporting members.

3. Filter (20) according to Claim 1 or 2, **characterized in that** the supporting member (21) has a cell density of from 100 to 1000 cpsi, preferably 150 to 500 cpsi.

4. Filter (20) according to any of Claims 1 to 3, **characterized in that**, under application conditions, it has a pressure drop of 10 mbar or less, preferably 5 mbar or less.

5. Filter (20) according to any of Claims 1 to 4, **characterized in that** the component for the physisorption is selected from the group consisting of activated carbon, bentonite, siliceous earths and zeolites; and/or **in that** the component for the chemisorption comprises peptides, proteins or keratin-containing fibers.

6. Filter (20) according to any of Claims 1 to 5, **characterized in that** the ionic liquids comprise cations selected from the group consisting of optionally alkylated imidazolium, pyridinium, pyrrolidinium, guanidinium, uronium, thiouronium, piperidinium, morpholinium, ammonium and phosphonium ions and anions selected from the group consisting of tetrafluoroborates, trifluoroacetates, triflates, hexafluorophosphates, phosphinates, tosylates, imides, amides, sulfates and halides.

7. Filter (20) according to any of Claims 1 to 6, **characterized in that** the filter layer (22) has a matrix, where constituents of the matrix preferably comprise components for the physiosorption and/or components for the chemisorption; wherein the matrix preferably comprises binders; wherein the binders are preferably selected from the group consisting of mannurone, gulurone, alginate and pectin salts.

8. Filter (20) according to Claim 7, **characterized in that** the matrix incorporates ionic liquids.

9. Method for producing a filter (20) according to any of Claims 1 to 8, **characterized by** the following steps:
c) provision of a supporting member (21),
d) coating of the surfaces of the supporting member (21) with the filter layer (22).

10. Method according to Claim 9, **characterized in that** the surfaces of the supporting member (21) are coated with a solution of constituents of the filter layer (22); wherein, preferably after the coating with a solution of constituents of the filter layer (22), a fixing takes place; wherein the fixing preferably takes place by solidifying a binder of the filter layer (22); wherein the solidification of the binder preferably takes place through a reaction by means of applying a further component.

11. Use of a filter (20) according to any of Claims 1 to 8 for the filtering of the respiratory air in modes of transport, in particular aircraft.

12. Use of a filter (20) according to any of Claims 1 to 8 for the filtering of the bleed air from the compressor of an aircraft engine.

13. Aircraft, **characterized in that** it has at least one filter (20) according to any of Claims 1 to 8 for the filtering of the respiratory air.

14. Aircraft according to Claim 13, **characterized in that** at least one filter (20) is arranged at one or more of the following installation sites (1, 2, 3) :
- between bleed air valve and cooling unit (8),
- between cooling unit and mixing unit (9),
- between mixing unit (9) and exit of the supply pipes (6) to cabin (4) and/or cockpit (5).

15. Aircraft according to Claim 14, **characterized in that** at least one filter (20) is arranged within the pressurized cabin before the exit of the supply pipes (6) to cabin (4) and/or cockpit (5).

## Revendications

1. Filtre (20) destiné à lier des éléments constitutifs d'un flux de gaz, comprenant un corps porteur (21), qui présente une densité cellulaire de 50 à 1 600 cpsi, et une couche filtrante (22) appliquée sur les surfaces du corps porteur (21), **caractérisé en ce que** la couche filtrante (22) comprend les composants suivants :
a) un composant destiné à la physisorption des éléments constitutifs,
b) un composant destiné à la chimisorption des éléments constitutifs,
c) un composant destiné à dissoudre les éléments constitutifs huileux qui contiennent des liquides ioniques.

2. Filtre (20) selon la revendication 1, **caractérisé en ce que** le corps porteur (21) est choisi dans le groupe composé des corps porteurs en céramique et des corps porteurs métalliques.

3. Filtre (20) selon la revendication 1 ou 2, **caractérisé en ce que** le corps porteur (21) présente une densité cellulaire de 100 à 1 000 cspi, de préférence de 150 à 500 cpsi.

4. Filtre (20) selon l'une des revendications 1 à 3, **caractérisé en ce que** dans les conditions d'utilisation, il présente une chute de pression inférieure ou égale à 10 mbar, de préférence inférieure ou égale à 5 mbar.

5. Filtre (20) selon l'une des revendications 1 à 4, **caractérisé en ce que** le composant destiné à la physisorption est choisi dans le groupe composé du charbon actif, de la bentonite, des terres à diatomées et des zéolithes ; et/ou **en ce que** le composant destiné à la chimisorption contient des peptides, des protéines ou des fibres contenant de la kératine.

6. Filtre (20) selon l'une des revendications 1 à 5, **caractérisé en ce que** les liquides ioniques contiennent des cations choisis dans le groupe composé des ions d'imidazole, de pyridinium, de pyrrolidinium, de guanidinium, d'uronium, de thiouronium, de pipéridine, de morpholinium, d'ammonium et de phosphonium, éventuellement alkylés, et d'anions choisis dans le groupe composé des tétrafluoroborates, des trifluoroacétates, des triflates, des hexafluorophosphates, des phosphinates, des tosylates, des imides, des amides, des sulfates des halogénures.

7. Filtre (20) selon l'une des revendications 1 à 6, **caractérisé en ce que** la couche filtrante (22) possède une matrice, les éléments constitutifs de la matrice comprenant de préférence des composants destinés à la physisorption et/ou des composants destinés à la chimisorption ; la matrice contenant de préférence des agents liants ; les agents liants étant de préférence choisis dans le groupe composé des sels mannuroniques, guluroniques, d'alginate et de pectine.

8. Filtre (20) selon la revendication 7, **caractérisé en ce que** la matrice inclut des liquides ioniques.

9. Procédé de fabrication d'un filtre (20) selon l'une des revendications 1 à 8, **caractérisé par** les étapes suivantes :
c) fourniture d'un corps porteur (21),
d) revêtement des surfaces du corps porteur (21) avec la couche filtrante (22).

10. Procédé selon la revendication 9, **caractérisé en ce que** les surfaces du corps porteur (21) sont revêtues d'une solution d'éléments constitutifs de la couche filtrante (22) ; une immobilisation ayant de préférence lieu après le revêtement avec une solution d'éléments constitutifs de la couche filtrante (22) ; l'immobilisation étant de préférence réalisée par durcissement d'un agent liant de la couche filtrante (22) ; le durcissement de l'agent liant étant de préférence réalisé par une réaction par application d'un composant supplémentaire.

11. Utilisation d'un filtre (20) selon l'une des revendications 1 à 8 pour le filtrage de l'air respiratoire dans des moyens de transport, notamment des aéronefs.

12. Utilisation d'un filtre (20) selon l'une des revendications 1 à 8 pour le filtrage de l'air de distribution issu du compresseur d'un groupe propulseur d'aéronef.

13. Aéronef, **caractérisé en ce qu'**il possède au moins un filtre (20) selon l'une des revendications 1 à 8 pour le filtrage de l'air respiratoire.

14. Aéronef selon la revendication 13, **caractérisé en ce qu'**au moins un filtre (20) est disposé au niveau d'un ou plusieurs des lieux d'installation (1, 2, 3) suivants :
- entre la vanne à air de distribution et une unité de refroidissement (8),
- entre une unité de refroidissement et de mélange (9),
- entre une unité de mélange (9) et la sortie des tubes d'alimentation (6) dans la cabine (4) et/ou le cockpit (5).

15. Aéronef selon la revendication 14, **caractérisé en ce qu'**au moins un filtre (20) est disposé à l'intérieur de la cabine pressurisée avant la sortie des tubes d'alimentation (6) dans la cabine (4) et/ou le cockpit (5) .
